# EUROPEAN PATENT APPLICATION

(11) **EP 0 795 760 A2**
(43) Date of publication of application: **17.09.1997**
(21) Application number: 97104278.3
(22) Date of filing: 13.03.1997
(51) Int. Cl.: G01S 5/14, A63B 29/02

(54) **Location search system**

(30) Priority: 14.03.1996 JP 57604/96
(71) Applicant: Akai, Koji, Kamakura-shi, Kanagawa-ken (JP)
(72) Inventor: Akasaka, Noboru, Tokyo (JP); Akai, Koji, Kamakura-shi, Kanagawa-ken (JP)
(74) Representative: Geyer, Ulrich F., Dr. Dipl.-Phys.

(57) **Abstract**

A location search system in accordance with the present invention comprises (1) a unit, which is carried by the searchee, having computing means responsive to GPS electromagnetic waves emitted from GPS satellites for computing geometrical data of the current location of the searchee and (2) a unit used by a searcher having radio transceiver means for transmitting data to and receiving data from the above unit on the searchee's side and display means for displaying the current location of the searchee on a map. By using this system, the searcher is able to readily and precisely specify the location of the searchee on a display screen. Further, the relative geometrical relationship including the direction and distance between the searchee and the searcher and, especially, such a relative geometrical relationship when the searcher is moving in a vehicle can, in order to further accelerate and improve the precision of the search of the missing person, be clarified on a map of the neighboring area.

## Description

The present invention relates to a location search system for a person which utilizes radio communication and, in particular, a location search system used for locating an elderly person who is moving around, a person who may suffer from a sudden physical disorder while away from home, a missing person in cases of natural disasters such as earthquakes, fire or flood, or a missing mountain climber. In one form of the invention, the location search system comprises a unit carried by or attached to a person being searched for, and a unit used by a person conducting a search, which is in a radio communication with the other unit.

In the prior art, no systems have existed that could be effectively used to locate an elderly person who is moving around or a person at potential physical risk and who may have a fit while being away from home. Nor have any effective systems existed that could be used to locate a missing person who may be buried in the case of, for example, an earthquake or otherwise be missing in a fire or flood, or for a missing climber.

In such cases, it is quite probable that the person who is missing and being searched for is in need for an emergency medical treatment and thus it is strongly desirable that the location of such a person should be quickly and precisely determined. Further, if medical center personnel can be informed remotely of a located person's physical data (vital signs) in advance, such knowledge will be of great help in preparing appropriate medical treatment in an ambulance or at a medical center. Such search and health determination operations could be critical in saving life. However, in the prior art, no such system has been developed.

A car navigation system may be thought of as being similar to a system of the present invention. In such a car navigation system, a car driver utilizes electromagnetic waves transmitted from GPS (Global Positioning System) satellites so that the driver can determine where he/she is located and proceed to a chosen destination. In addition, explorers traversing polar regions where no visual landmarks are available use a similar location recognition system which is based on radio transmission with satellites.

However, the present invention relates to a system in which the person to be located is no longer capable of identifying the location him/herself by sending a signal and thus a third party uses this search system to rescue the person. Thus the system of the present invention differs from a car navigation system in its objectives. Technically speaking, the location search system in accordance with the present invention does not comprise a bilateral communication system between a GPS satellite (inclusive of the control center on the ground which constitutes the entire GPS system) and the unit on the receiving party but conducts a location search by using the unit mounted on or carried by the missing person to be located as a relay station. Therefore, the system of the present invention differs from the above-mentioned prior art systems.

One purpose of the present invention is to provide a system by which a current location of a person to be searched for can be readily and remotely determined by using radio communication without any time restriction.

Another purpose of the present invention is to provide a location search system by which the person to be searched for can be followed on a map by determining the relative geometrical relationship including the direction and distance on the map in specifying the current location of the person to be searched for.

A further purpose of the present invention is to provide a system by which a predetermined set of measured physical data can be remotely acquired in advance, in addition to determining the current location of the person.

A location search system in accordance with the present invention comprises (1) a unit, which is carried by or attached to the searchee, having computing means responsive to GPS electromagnetic waves emitted from GPS satellites for computing geometrical data of the current location of the searchee and (2) a unit used by a searcher having radio transceiver means for transmitting data to and receiving data from the above unit carried by the searches and display means for displaying the current location or the searchee on a map.

By using the location search system of the present invention, the searcher is able to readily and precisely specify the location of the searchee on a display screen. Further, in accordance with one aspect of the invention, the relative geometrical relationship including the direction and distance between the searchee and the searcher and, especially, such a relative geometrical relationship when the searcher is moving in an automobile or any other vehicle can, in order to further accelerate and improve the precision of the search of the missing person, be clarified on a map.

Further features of the present invention will become apparent to those skilled in the art to which the present invention relates from reading the following detailed description with reference to the accompanying drawings, in which:
Fig. 1 is a perspective view of a location search system in accordance with the invention.
Fig. 2 schematically shows the internal configurations of a unit carried by the searchee and a unit used by the searcher.

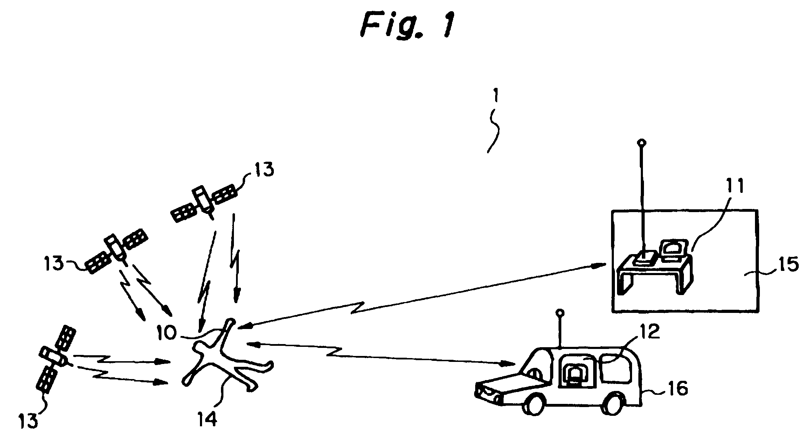

By referring to the attached drawings, embodiments of the invention will be described. Fig. 1 illustrates a perspective view of the location search system 01 according to the invention. The unit 10 is normally worn or carried by the searchee on the body, for example around the wrist. The unit 11 or 12 used by the searcher may be set up at a local medical center 15 in the area (unit 11) or may be carried by the searcher or installed in an automobile 16 (unit 12).

The unit 10 is responsive to radio transmission conducted via electromagnetic waves emitted from GPS satellites to register its own location and transmits a signal representative of location data. The mechanism of this location search is based on the Global Positioning System (GPS) which is well known to those skilled in the art, wherein the location is determined by measuring the difference in time required for the electromagnetic waves from at least three satellites to reach a searchee. If electromagnetic waves from four or more satellites are utilized, a location in three dimensions can be determined.

Unit 11 or 12 on the searcher's side receives signals from unit 10 carried by the searchee and displays the current location of the searcher together with map data in the neighboring area thereof. Such map data may be stored in an external Storage unit 26 of unit 11 or 12 used by the searcher. More particularly, geometrical data stored in a storage means such as a CD-ROM used in a CD-ROM drive provided in the unit may be, in response to a requested search area, read out. Further, unit 11 or 12 on the side of the searcher is responsive to electromagnetic waves transmitted from unit 10 carried by the searchee for detecting and displaying a direction and distance to the current location of the searchee relative to the searcher, to facilitate searching. This function is intended to clarify the relative geometrical relationship between the searchee and the searcher particularly when the unit used by the searcher is installed in a moving automobile 16 as unit 12.

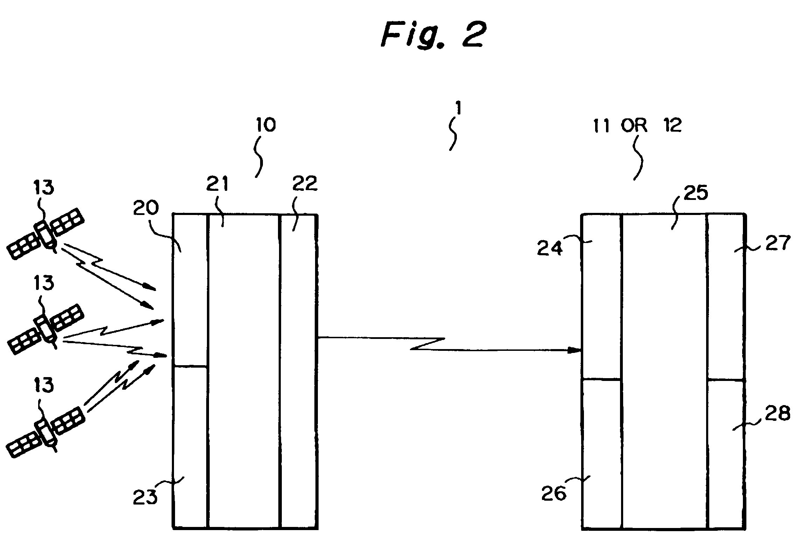

Fig. 2 schematically illustrates the internal configurations of unit 10 carried by the searchee and unit 11 or 12 used by the searcher, both of a location search system in accordance with the present invention. As shown in the figure, unit 10 on the searchee's side receives electromagnetic waves from at least three GPS satellites at the receiver 20 of the GPS electromagnetic waves, the central processing unit (CPU) 21 calculates on the basis thereof the data in three dimensions on the location of the searchee, and the associated location information of the searchee is transmitted to unit 11 or 12 on the searcher's side.

Unit 11 or 12 used by the searcher receives location information from unit 10 carried by the searchee at transceiver 24, processes the information at CPU 25, and displays on the display screen 27 the relative geometrical relationship between the searcher and the searchee. It is also possible to output audio guidance from on audio output 28. A geometrical map database is stored in storage 26 associated with unit 11 to 12 on the searcher's side. Geometrical data corresponding to each area and stored in a CD-ROM may be read out therefrom using the CD-ROM drive and displayed on display screen 27. The searcher may then look at display screen 27 to determine the searchee's location.

When the location search system of the present invention is installed in and moves together with a vehicle such as an automobile or airplane to search for a missing person, unit 12 in the automobile receives electromagnetic waves transmitted from unit 10 on the searchee's side at radio receiver 24, calculates the direction, distance, and region of the transmission from the information included therein, and outputs the result on display screen 27 or via audio output 28 so that recovery can be effected. By using the system installed in a moving automobile in this manner, the searcher is able to conduct a quick and precise search even if the relative geometrical relationship between the searcher and searchee constantly varies in a certain area. An example of such a case would be when an elderly person suffering from poriomania is to be searched for.

Further, the location search system may be equipped with means for monitoring the physical condition of the searchee by continuously measuring and acquiring medical data (vital signs) of the searchee. Referring to Fig. 2, unit 10 carried by the searchee receives vital signs such as a pulse wave, pulse, electrocardiogram, and SpO2 at data input 23, which are continuously or intermittently detected, processes such data at CPU 21, and transmits the results to unit 11 or 12 via radio transceiver 22. Unit 11 or 12 used by the searcher receives such data at radio transceiver 24, interprets it at CPU 25, and outputs the results on display screen 27 or from audio output 28 to inform the searcher of the medical status of the searchee.

In unit 11 or 12 on the searcher's side, when CPU 25 determines that the medical condition assumed from the vital signs of the searchee is not within an acceptable threshold range associated with the person, which threshold range has been predetermined by a physician in charge, and that the searchee may be in a critical condition. CPU 25 has audio output 28 output an alarm sound indicating that an especially urgent search is required. By this function, the system can inform medical personnel at a medical center 15 associated with the searcher of the emergent situation and have them prepare for the arrival of the located person in advance.

Unit 10 carried by the searchee may preferably be manufactured as a small and light device to be carried without imposing any substantial burden. A light power supply such am a lithium battery may be used in unit 10. In addition, how often transmission between the searcher and the searchee is conducted can be controlled by a control signal transmitted from unit 11 or 12 on the searcher's side in order to save power consumption. That is, instead of continuously transmitting signals to unit 11 or 12 on the searcher's side from unit 10 on the searches's side, control signals may be transmitted once every several minutes. Also, a location search may be commenced at a certain moment and terminated at a certain moment. The control signals may monitor the normal operation of unit 10 on the searchee's side.

As described above, the location search system of the present invention makes it possible to readily search for a milling person. This system is particularly effective for locating an elderly person who is moving around, a person who may suffer from a sudden physical disorder and who is away from home, a missing person in the case of a natural disaster such as an earthquake, fire, or flood, or a missing mountain climber.

Further, by using a function of monitoring the searches's physical condition through the detection of his/her vital signs, this system plays a critical role in determining the medical condition of a person to be searched for, thereby being effective in emergent situations.

From the above description of the invention, those skilled in the art will perceive improvements, changes, and modifications. Such improvements, changes, and modifications within the skill of the art are intended to be covered by the appended claims.

The present invention relates to that disclosed in Japanese Patent Application No. 57,604/1996 made by the same inventors, the entire disclosure of which is herein incorporated by reference.

## Claims

1. A location search system for remotely specifying a current location of a searchee, the system comprising:
a unit carried by the searchee comprising
means for receiving electromagnetic waves from a plurality of artificial satellites which constitute the Global Positioning System (GPS),
means for calculating the current location of the searchee on the ground on the basis of the time required for the electromagnetic waves to reach the unit, and
means for transmitting signal that includes the calculated location information, and
a unit used by a searcher comprising
means for receiving the signal from the unit carried by the searchee,
a geometrical database in which geometrical map information of each area is stored, and
display means for displaying the current location of the searchee together with a map of the neighboring area of the location read out from the database.

2. The location search system recited by claim 1 wherein the current locations of one or more searchees can be searched for and displayed.

3. The location search system recited by claim 1 wherein a plurality of searchees can be located with the electromagnetic waves having a single frequency by associating an identification (ID) number with each of the plurality of searchees in transmitting the location information.

4. The location search system recited by claim 1 wherein the unit used by the searcher further comprises means for following the searchee by responding the signal transmitted by the unit carried by the searchee for detecting and displaying the direction in which the signal is transmitted.

5. The location search system recited by claim 4 further comprising means for measuring and displaying the distance between the searcher and the searchee on the basis of the time required for the signal to reach the unit used by the searcher.

6. The location search system recited by claim 1 wherein
the unit carried by the searchee further comprises means for monitoring information concerning the physical condition of the searchee based on physical data (vital signs) of the searchee detected on the real time basis,
the transmission means of the unit carried by the searchee transmits the monitored physical condition information together with the location information, and
the unit used by the searcher generates an alarm signal when it is determined that the searchee is in a medically critical condition by comparing the monitored physical condition with the reference data associated with the specific searchee stored in a database.

7. The location search system recited by claim 1 the unit used by the searcher further comprises means for receiving the electromagnetic waves transmitted by the unit carried by the searchee with a group of electromagnetic wave sensitive elements and means responsive to the electromagnetic waves for detecting the location from which the electromagnetic waves are being emitted and displaying the location on the display screen.

8. The location search system recited by claim 1 wherein the unit carried by the searchee further comprises means for receiving control signal from the unit used by the searcher and means responsive to the control signal for controlling the operation of each of the functional means.

9. The location search system recited by claim 1 wherein the unit carried by the searchee is responsive to the control signal from the unit used by the searcher for transmitting the signal including the location information in such a manner that the transmission commences at a predetermined time and ends at a predetermined time after a desired time interval.

10. A location search system for remotely specifying a current location of a searchee, the system comprising:
a unit carried by the searchee comprising
means for receiving electromagnetic waves from a plurality of artificial satellites which constitute the Global Positioning System (GPS),
means for calculating the current location of the searchee on the ground on the basis of the time required for the electromagnetic waves to reach the unit, and
means for transmitting signal that includes the calculated location information, and
a unit used by a searcher comprising
means for receiving the signal from the unit carried by the searchee,
a geometrical database in which geometrical map information of each area is stored, and
display means for displaying the current location of the searchee together with a map of the neighboring area of the location read from the database, wherein
the unit carried by the searchee further comprises means for monitoring information concerning the physical condition of the searchee based of physical data (vital signs) of the searchee detected on the real time basis,
the transmission means of the unit carried by the searchee transmits the monitored physical condition information together with the location information, and
the unit used by the searcher generates an alarm signal when it is determined that the searchee is in a medically critical condition by comparing the monitored physical condition with the reference data associated with the specific searchee stored in a database.
